# EUROPEAN PATENT APPLICATION

(11) **EP 4 542 202 A1**
(43) Date of publication of application: **23.04.2025**
(21) Application number: 23823317.5
(22) Date of filing: 06.06.2023
(51) Int. Cl.: G01N 21/01, G01N 21/25, G01N 21/69

(54) **MOLECULAR SPECTROSCOPY EQUIPMENT WITH A BROAD SCANNING RANGE FOR CONTINUOUSLY CHARACTERISING THE POLLUTANT LOAD OF WASTE WATER**

(30) Priority: 16.06.2022 ES 202230530
(71) Applicant: Universidad Politecnica de Cartagena, 30202 Cartagena (Murcia) (ES)
(72) Inventor: CARRERES PRIETO, Daniel, 30202 Cartagena (MURCIA) (ES); CERDÁN CARTAGENA, José Fernando, 30202 Cartagena (MURCIA) (ES); GARCÍA BERMEJO, Juan Tomás, 30202 Cartagena (MURCIA) (ES); SUARDIAZ MURO, Juan, 30202 Cartagena (MURCIA) (ES)
(74) Representative: Pons
(86) International application number: PCT/ES2023/070375
(87) International publication number: WO 2023/242456

(57) **Abstract**

The present invention relates to equipment comprising an assembly for storing and analysing samples over time that comprises a rotating and removable loader (11) housing containers (12) for storing and analysing the samples; a suction assembly (26) for taking samples that is equipped with a primary sensor (31); lifting means (34) for lifting the suction assembly (26); a multispectral analysis unit (18); means for injecting the samples taken; and positioning means for positioning a secondary sensor (44) responsible for characterising the stored samples. The equipment allows the samples to not just be taken and characterised automatically at the time they are taken, but also provides the option of characterising the sample over time, which results in a broadening of the parameters to be determined, such as the size and mass concentration of the sedimentable and non-sedimentable particles present in the sample.

## Description

### OBJECT OF THE INVENTION

The object of the present invention, as established by the title of the invention, is a molecular spectroscopy equipment with a broad scanning range for continuously characterising the pollutant load of waste water, including characterising the size and mass concentration of particles, which allows storing and analysing the samples taken over time, which results in a better characterisation of the samples.

The equipment object of the invention can also carry out the characterisation of the size and mass concentration of the sedimentable and non-sedimentable particles present in the sample.

The present invention is characterised by the special design and configuration of each and every one of the parts that make up the characterisation equipment, such that an autonomous, low-cost, low-consumption and low-dimension equipment is achieved, which can be implemented at any point in the sanitation network, which is capable of acquiring and characterising waste water without human intervention and without the need to subject the samples to pretreatment or chemical reagents. The system is also capable of transmitting the analysis results to the cloud as well as storing the desired samples for later comparison in the laboratory.

The equipment can also analyse samples stored in containers that are manually introduced into the tray, as if it were laboratory equipment.

The samples can be those stored by the equipment itself, as well as those that the user has manually introduced into the rotating loader.

The invention makes use of variable wavelength spectrophotometry to carry out the water characterisation process, based on mathematical estimation models.

Multispectral analysis is performed using a rotating disc composed of multiple LED diodes arranged concentrically that are aligned with the waste water sample being studied. This sample is taken automatically at regular time intervals and/or upon user request by means of a pump that circulates the water through an open circuit. Transversely to the circuit, a suction cylinder coupled to a motorised system sucks and stores the sample under study therein. The sample, after being analysed, can be evacuated through the equipment's outlet channel, or stored in containers arranged in a removable rotating circular loader that is aligned with an injection mechanism. This sample loader can be removed using a tray located at the front portion of the equipment.

Therefore, the present invention is limited to the scope of devices or equipment for spectroscopic characterisation of water samples, specifically, in the present invention, of waste water samples.

### BACKGROUND OF THE INVENTION

In the state of the art, utility model ES1273069U is known, which discloses an equipment for the characterisation of the pollutant load present in waste water, comprising a rotating wavelength selector disc that internally comprises a disc with a plurality of diodes, wherein this disc comprises a self-positioning system, it further comprises a vertical displacement mechanism, and a tower comprising a vertical cavity, a circular channel where at one of its ends it is provided with an opening coinciding with a through hole in the circular support that houses a diode.

Although the equipment fulfils the functionality for which it is designed, which is spectroscopic characterisation, there are aspects that can be improved, since it does not allow storing the samples and subsequent analysis thereof over time so that an analysis can be carried out at different time intervals, which allows for a better characterisation as a continuous analysis of the samples is being performed.

The equipment disclosed in utility model ES1273069U does not characterise water pollution by size and mass concentration, lacks a rotating and removable loader for housing samples, lacks a suction assembly for taking samples with its corresponding lifting means, lacks means for injecting the samples, lacks means for rotating and lifting the multispectral analysis unit and lacks a secondary sensor with positioning means, as the equipment subject to this application does have.

All these differences have technical effects such as:
- classifying pollutants,
- removing/inserting samples, taking samples,
- storing samples, analysing a stored sample, etc.),
which solve technical problems such as:
- better characterising the pollutant particles,
- facilitating the introduction and extraction of samples,
- automating sample taking,
- storing samples,
- analysing the same sample at different points in time, etc.

All of the above results in a better and more automated operation of the machine that is the object of our invention.

Other documents related to the subject of the application would be:
- Document CN109374555A which discloses a spectroscopy equipment for analysing liquids such as water, that comprises, among other elements, a rotating disc with LEDs and a horizontal rotation axis, a rotating circular table with a vertical rotation axis and holes for holding the samples to be analysed and a photodiode in the centre, and a series of drives.
- Document CN212722596U refers to an online equipment for analysing waste water using ultraviolet spectroscopy.
- Document CN216386768U discloses a spectroscopy equipment for multispectral analysis of waste water.

All the above equipment in general lacks a suction assembly, associated with a primary sensor; lifting means for lifting the suction assembly; positioning means for positioning a secondary sensor mounted on the main block for taking samples; means for injecting the samples taken arranged on the main support platform that manage to solve the technical problems indicated for document ES1273069U.

Therefore, the object of the present invention is to improve the stated aspects by developing equipment such as that described below and essentially included in the first claim, and which allows the automatic taking, storing and subsequent analysis of samples over time.

### DESCRIPTION OF THE INVENTION

The object of the present invention is essentially included in the independent claim and the different embodiments are included in the dependent claims.

The present invention relates to a device capable of analysing and characterising the pollutant load of waste water in real time without the need for human intervention or subjecting the samples to chemical reagents or pretreatment.

This characterisation is carried out by means of a multispectral analysis of the samples, using LED technology, which allows costs, dimensions and consumption of the equipment to be significant reduced. Furthermore, since the equipment stores the sample during the analysis, this allows the study of the evolution of the multispectral analysis of the same sample over time, which allows the indirect characterisation of the sedimentable and non-sedimentable suspended solids by defining both their size and the concentration associated with said size in the waste water sample.

To date, there is no equipment on the market that indirectly determines the size and quantity of solids in a waste water sample based on the temporal variation of the response using molecular spectroscopy equipment with a broad scanning range.

The equipment is designed to operate in waste water treatment plants and at points in the sanitation network, and does not require to be located inside the plant. The system pumps the water to be analysed using a pump installed inside the equipment, and makes the water pass through an open circuit until it is evacuated through another pipeline. This recirculation helps to prevent obstruction of the internal pipes and to constantly renew the water to be analysed.

At the central point of the pipe circuit, a sample is taken by means of a motorised suction cylinder while the water continues to circulate inside the equipment, in order to avoid the formation of air bubbles, as well as obstructions during the acquisition.

Before starting taking samples, the pump circulates the water through the internal conduits of the equipment for a certain time, to avoid analysing the water from the previous cycle.

Once the water to be analysed has been stored in the suction cylinder, the equipment performs a multispectral analysis using a motorised rotating disc composed of multiple LEDs radially and concentrically arranged, which are aligned with the sample until they reach the sensor, consisting of a photodiode with a broad range. Internally, by means of a mathematical algorithm, the spectral response produced by the different wavelengths emitted by each LED is simultaneously broken down, so that a certain region of the range can be analysed with just a few diodes.

Once the spectral response of the sample has been obtained, the equipment transmits the results to a server using different communication protocols where, by means of mathematical algorithms, it is possible to automatically estimate various pollutant parameters related to water quality. To characterise the particle size, as well as its associated concentration, successive analyses will be carried out over different time intervals on the same sample. In this way, the equipment takes a sample, performs its analysis on said specific sample and repeats the analysis on said sample at spaced periods of time. The variation in the transmittance measured over the different time intervals allows indirect characterisation of the size and concentration of sedimentable and non-sedimentable suspended matter over the measurement intervals.

Once the sample has been analysed, the equipment object of the invention can perform two options:
- Evacuate the water through the outlet channel, by means of the movement of the piston of the suction cylinder, which cleans the walls during its travel, leaving the analysis unit ready for the next measurement.
- Or it can store the sample in glass containers, contained in a rotating loader that is placed on a removable tray, located at the front portion of the equipment, by means of an automatic injection mechanism. This consists of a needle that moves vertically to introduce the water contained in the suction cylinder into the containers, where once filled, the injector rises and the loader rotates until the next container is aligned with the injection mechanism, leaving the system ready for the next storage cycle. The equipment object of the invention has the option of re-analysing these stored samples, by means of a second retractable sensor block, which is deployed during the analysis and retracted when the user wishes to remove the tray containing the samples. The multispectral analysis unit, the main element of which is a disc with LED diodes, rotates 180° to align the first diode with the container to be analysed, being able to transmit the result of the analysis to the cloud at the end of the analysis.

Periodically, the equipment performs a calibration process by taking distilled water from a tank inside the equipment. By means of a three-way solenoid valve, the connection between the suction cylinder and the internal circulation channel containing the waste water being pumped is changed to a connection between the suction cylinder and the container for distilled water. The suction cylinder is completely filled with distilled water by means of a motorised system, which is then evacuated through the outlet channel after switching the solenoid valve, in order to purge any air that may be trapped in the system, as well as to clean the analysis unit more thoroughly. After this cleaning, the equipment switches the solenoid valve again to take a new sample of distilled water, which it will use for calibration. To do this, it aligns each of the LEDs on the multispectral analysis unit with the suction cylinder and stores the intensity level recorded for each of them. After the calibration process, the equipment switches the solenoid valve again to be able to evacuate the distilled water through the outlet channel.

The spectroscopy equipment object of the invention comprises:
- a first part arranged at the lower portion of the equipment and intended for the water pumping means,
- a main support platform which is secured to the side walls of the equipment and which divides the interior space above the means intended for pumping water into a lower portion and an upper portion,
- an assembly for storing and analysing samples over time housed in the lower space defined under the main support platform and attached to the side walls of the equipment and comprising a rotating and removable loader housing containers for storing and analysing the samples, where this rotating and removable loader is provided with extraction and rotation means,
- a suction assembly for taking samples mounted on a main block for taking samples on which a primary sensor is arranged, the main block being attached on the main support platform
- lifting means for lifting the suction assembly for taking samples arranged on the main block for taking samples.
- a multispectral analysis unit arranged opposite to the rotating and removable loader and with the suction assembly for taking samples, where said multispectral analysis unit is equipped with means that enable both a rotation and a lifting and lowering movement,
- means for injecting the samples taken arranged on the main support platform
- positioning means for positioning a secondary sensor mounted on the main block for taking samples.
- an electrical panel intended to support all the control and power supply means of the equipment.

All the elements described above allow the samples to not just be taken and characterised automatically at the time they are taken, but also provides the option of characterising the sample over time, which results in a more precise characterisation. To this end, the equipment is provided with a rotating and removable loader, a primary sensor for characterising the sample at the time of extraction and a secondary sensor for characterising the sample already stored in one of the containers of the rotating loader.

The means for characterisation at the time of extraction are provided with a suction assembly with lifting means for lifting the suction assembly, associated with means for injecting the samples taken.

The equipment object of the invention can analyse the temporal evolution of the samples taken, both with the main analysis block and by means of the storage tanks housed therein, which allow collecting samples in a much shorter period of time than in the main block, to subsequently analyse the same by means of an associated secondary sensor, with lifting and lowering means that allow its positioning in front of the sample taken in combination with the rotation of the rotating loader.

Samples can also be stored and analysed from the containers (12) by means of the secondary sensor and the wavelength selector disc which rotates 180° to align the first LED on the selector disc with the sample stored in the container (12).

The distance between the secondary sensor or containers (12) and the main samples in the suction cylinder (27) or the primary sensor is a distance equal to the outer diameter of the wavelength selector disc. This allows taking, storing and characterising the stored samples in a shorter period of time than that in the aspiration cylinder (27) by aligning the wavelength selector disc (19) in a 180° rotation.

Said distance, equal to the external diameter of the wavelength selector disc (19), makes it possible to reproduce the same measurement conditions between the two possible measurement methods provided in the equipment. Since for both forms of measurement, both through the primary sensor and cylinder (27) and through the secondary sensor and containers (12), the equipment is able to analyse the particular size from the variation of the spectral response as the particles sediment over time, on the same sample stored in the syringe.

The ability to characterise the samples over time allows to indirectly obtain values such as the size of solids and sediments, total suspended solids, the amount of total phosphorus and nitrogen, nitrogen in the form of nitrate, chemical oxygen demand (COD), as well as the biochemical oxygen demand after 5 days (BOD5), among others. All these values and parameters obtained allow a better characterisation of the samples.

Except when indicated otherwise, all of the technical and scientific elements used in this specification have the meaning commonly understood by a person with average skill in the art to which this invention belongs. When this invention is put into practice, methods and materials may be used that are similar or equivalent to the ones described in the specification.

Throughout the description and the claims, the word "comprise" and its variants are not intended to exclude other technical features, additions, components or steps. For those skilled in the art, other objects, advantages and features of the invention will be deduced from both the description and the practical use of the invention.

### DESCRIPTION OF THE FIGURES

As a complement to the description provided herein, and for the purpose of helping to make the features of the invention more readily understandable, in accordance with a preferred practical exemplary embodiment thereof, said description is accompanied by a set of drawings which, by way of illustration and not limitation, represent the following.
Figure 1 shows a perspective view of the equipment.
Figure 2 shows an exploded representation of the rotating and removable loader for storing and analysing samples.
Figure 3 shows a rear view of the inside of the equipment where the elements and portions making it up can be viewed.
Figure 4 shows a front view of the inside of the equipment where the casing has been removed for a better appreciation of the inside of the equipment.
Figure 5 shows an exploded perspective view of the analysis mechanism of the multispectral unit.
Figure 6 shows a perspective view of the industrial suction cylinder.
Figure 7 shows a first detail where, among other elements, the lifting system of the mechanism for taking samples can be observed.
Figure 8 shows a side view of the equipment where other details of the lifting mechanism for taking samples can be observed.
Figure 9 shows an almost upper view of the interior of the equipment where, in addition to the lifting system for lifting the mechanism for taking samples, a first and second servo can be observed.
Figure 10 is a detail of the machine, showing the location of the primary sensor and secondary sensor.
Figure 11 shows a detailed view of the interior of the machine where the location of the secondary sensor, among other elements, can be observed.

### PREFERRED EMBODIMENT OF THE INVENTION

In light of the figures, a preferred embodiment of the proposed invention is described below.

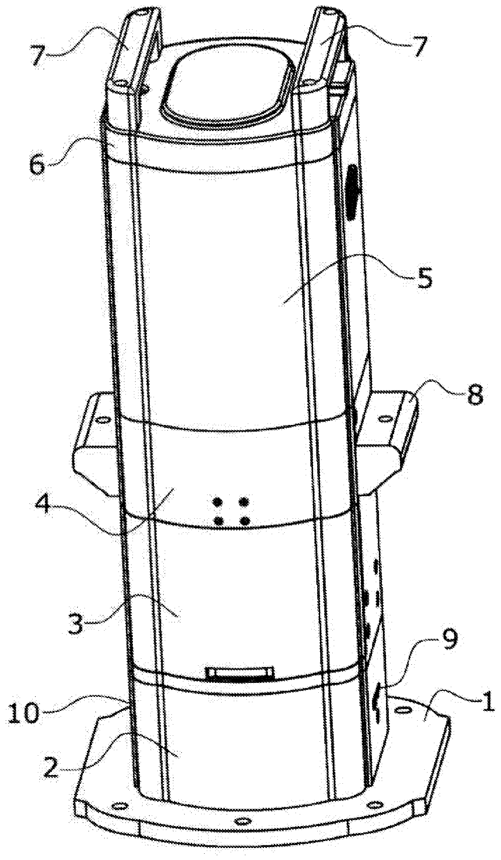

Figure 1 shows how the equipment object of the invention comprises a ground anchoring platform (1) on which an outer protective casing raises, divided into different portions or sections that can be opened and removed in some cases, a first portion (2) being observed intended for the water pumping means, followed by a second portion (3) housing an assembly for storing and analysing samples over time, followed by a third portion (4) equipped with a supplementary front cover that continues with a fourth portion (5) housing, among other elements, the control electronics and finished off by an upper cover (6) equipped with upper handles (7). The equipment is laterally provided with intermediate handles (8) approximately at the middle point of its height. On one side of the equipment, as shown, there is a water outlet channel (9), while on the other side there is a water inlet channel (10).

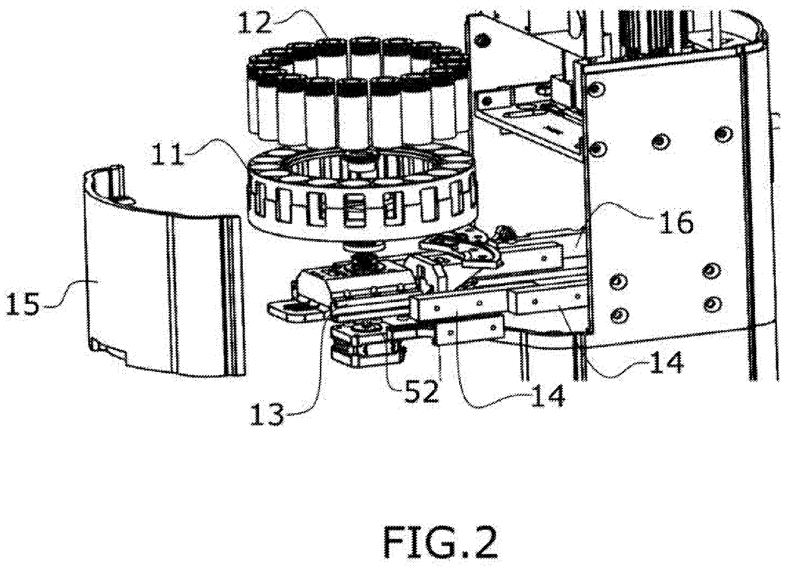

Figure 2 shows an exploded view of an assembly for storing and analysing samples over time, which comprises a rotating and removable loader (11) housing containers (12) for storing and analysing the samples, said rotating and removable loader is mounted on a removable cart (13) that runs on a removable platform by means of telescopic rails (14) that are secured to anchors (16) attached to the side walls of the interior of the equipment.

The rotating and removable loader can hold samples with variable sizes and different volumes depending on the user's requirements.

It is important to emphasize that this loader not only serves to store the containers (12) of the samples housed therein, but the same can also be analysed, once stored, over time, which allows a better characterising of the samples taken.

Under the removable cart (13) which houses a motor therein, not shown, the shaft (52) emerging from the same can be observed. The entire assembly for storing and analysing samples is housed inside the equipment and covered by a removable lid (15).

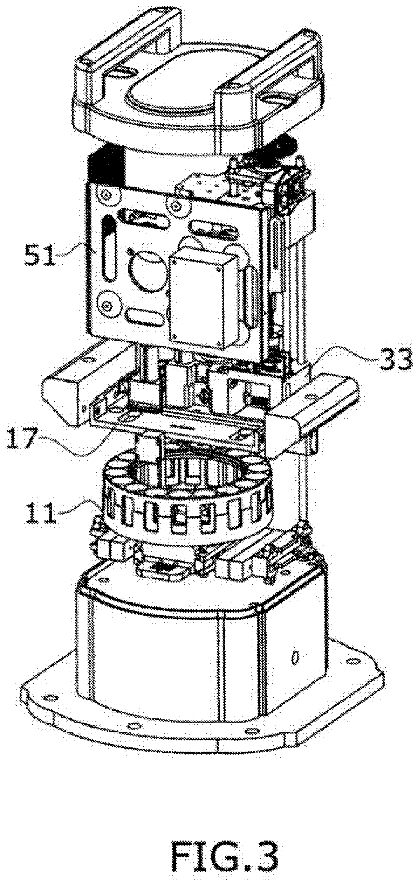

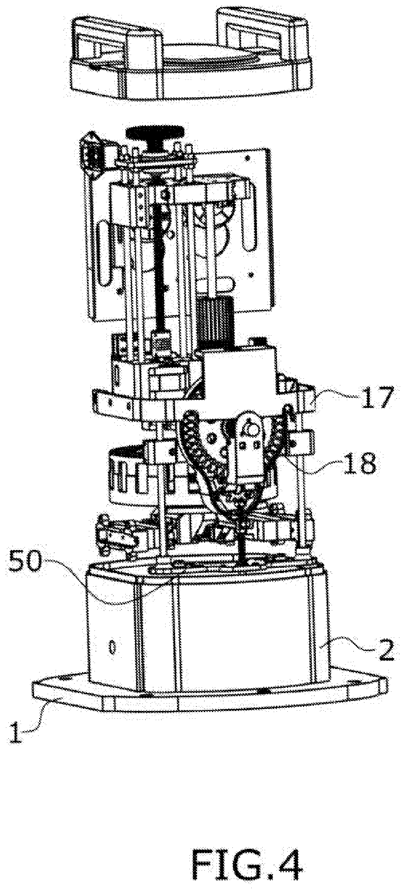

Figures 3 and 4 show the following key elements of the equipment: on the one hand, a main support platform (17) that is secured to the side walls of the equipment and on which a main block (33) for taking samples is attached. An electronic panel (51) intended to support all the control and power supply means of the equipment can also be observed.

Figure 4 shows the multispectral analysis unit (18) which is mounted on the first portion (2) intended for the pumping means.

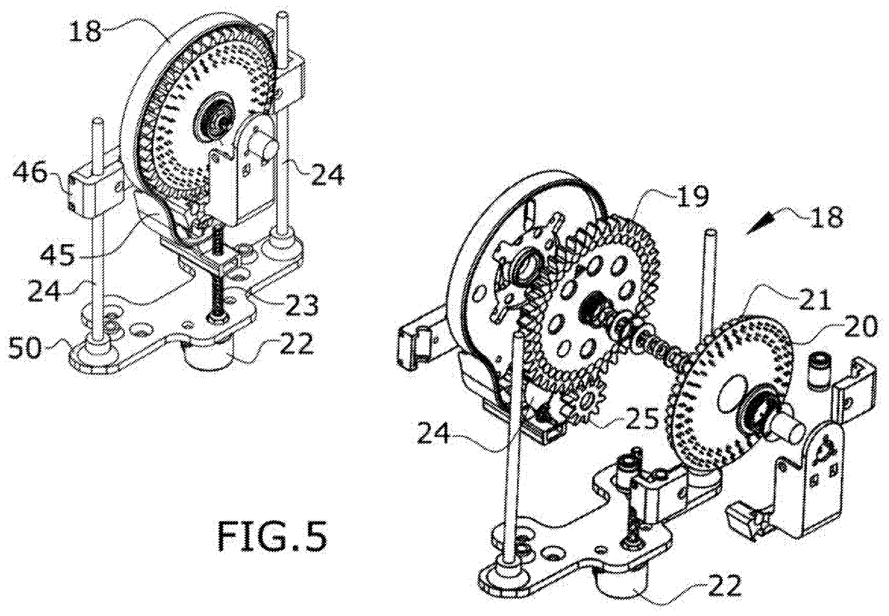

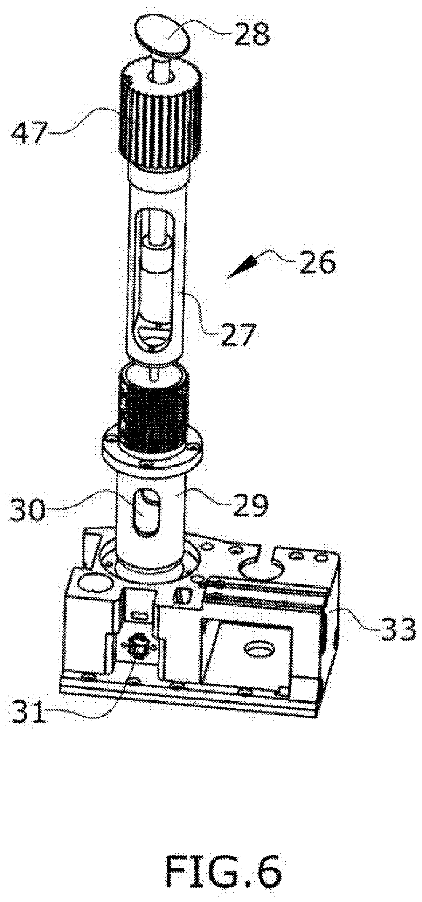

Figure 5 shows the elements that form part of the multispectral analysis unit (18) in detail, which, as can be seen, comprises a wavelength selector disc (19) which is a gear and on which is arranged a support plate (20) on which a plurality of LED diodes (21) are mounted such that the assembly rotates, producing the alignment of each LED diode (21) with the sample located in the suction cylinder (27) (figure 6).

The multispectral analysis unit (18) is equipped with two movements, a first lifting and lowering movement and a second rotation movement of the wavelength selector disc (19), wherein the first movement is achieved by means of a lifting and lowering motor (22), which is mounted on a support plate (50), wherein said motor rotates a threaded rod (23) which in its coupling with a support (45) of the wavelength selector disc (19) translates the rotation of the threaded rod (23) into a lifting and lowering movement. Said lifting and lowering movement is guided by means of vertical rods (24) on which slides (46) of the support (45) run, wherein said vertical rods (24) are attached to the lower side of the support plate (50) and at the upper side on the main support platform (17) (figure 4).

The lifting and lowering movement of the wavelength selector disc (19) allows the positioning of the LEDs mounted on said disc because it has several crowns of LEDs in a concentrical arrangement, which results in a greater number of wavelengths that can be emitted.

The rotation movement of the wavelength selector disc (19) is achieved by means of a motor (24) that has a pinion (25) coupled to its shaft that meshes with the wavelength selector disc (19).

Figure 6 shows the features of the suction assembly (26) which, as can be seen, comprises a suction cylinder (27) inside of which a transparent glass cylinder is arranged which is provided in its upper portion with a grooved upper cover (47) and on which is arranged a handle (28). The suction cylinder (27) can be housed in an outer jacket (29) which is equipped with a window (30), said outer jacket (29) being housed within the main block (33) for taking samples so that the window (30) is aligned with the location of the primary sensor (31), a location being observed on the main block (33) for taking samples to house a lifting motor (32) that vertically moves the rod of the suction cylinder (27).

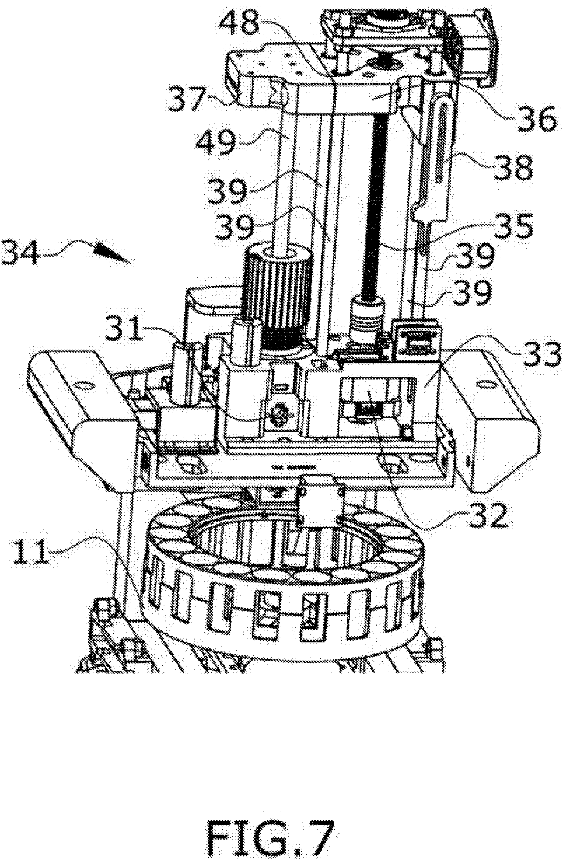

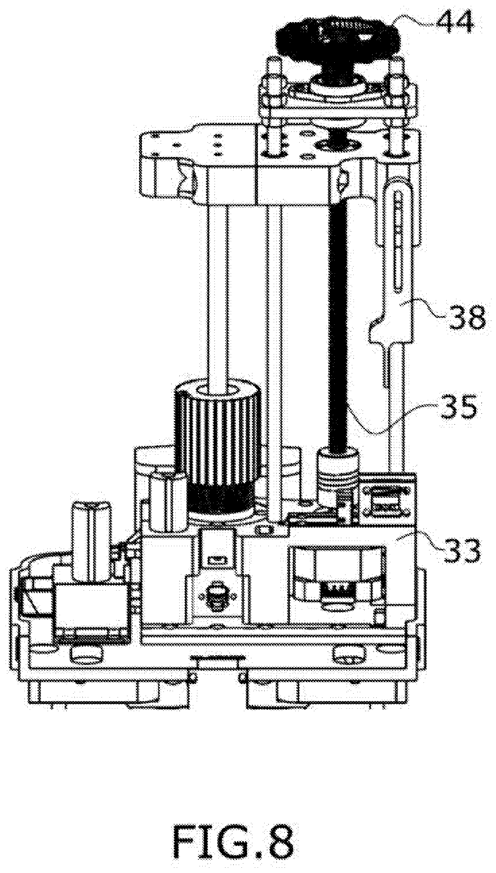

Figures 7 and 8 show the lifting and lowering means (34) of the mechanism for taking samples which, as can be seen, comprises a spindle (35) driven by the lifting motor (32), said spindle (35) being connected to a lifting platform (36) by means of a nut (48) which causes the transformation of the rotation of the spindle (35) into a vertical movement. Said lifting platform (36) comprises a connector (37) with the plunger (49) of the suction assembly through the handle (28).

The vertical movement of the lifting platform (36) is limited by a limit stop (38) and guided by means of guide rods (39) that are connected at their lower end to the main block (33) for taking samples and at their upper end pass through the lifting platform (36).

Figure 8 it shows that the spindle (35) can be manually driven by means of a handle (44).

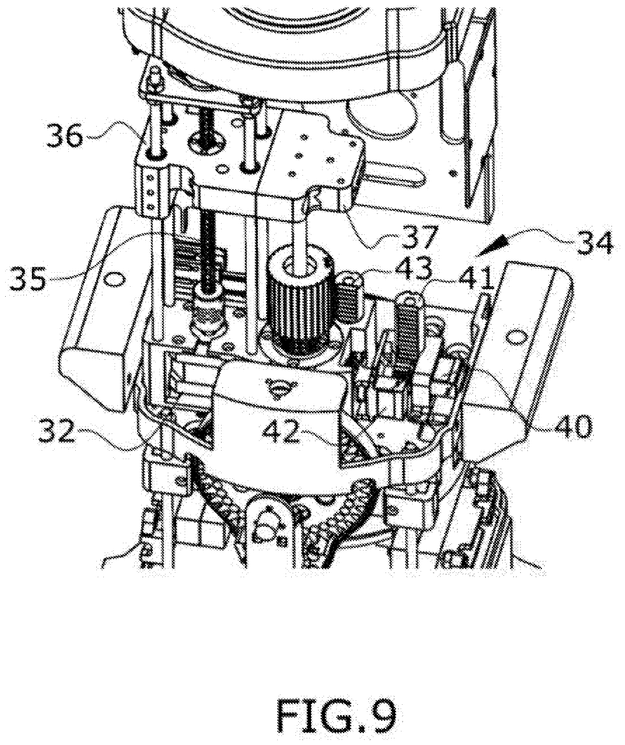

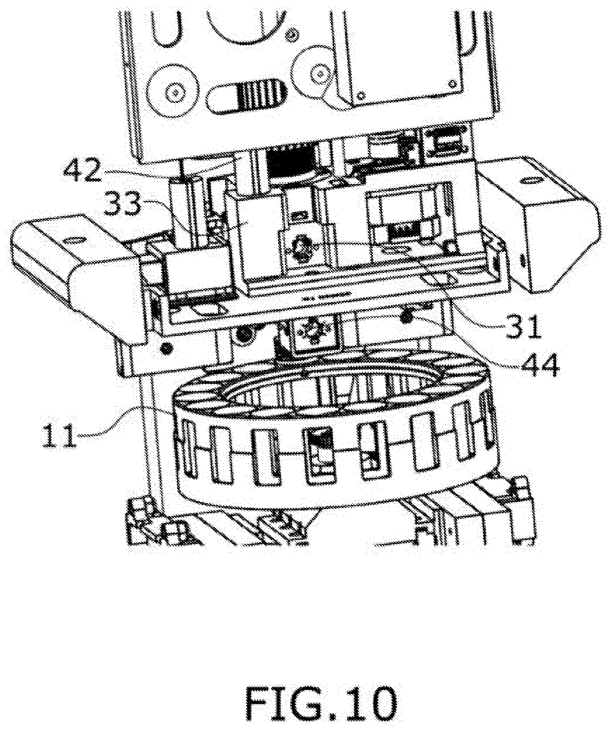

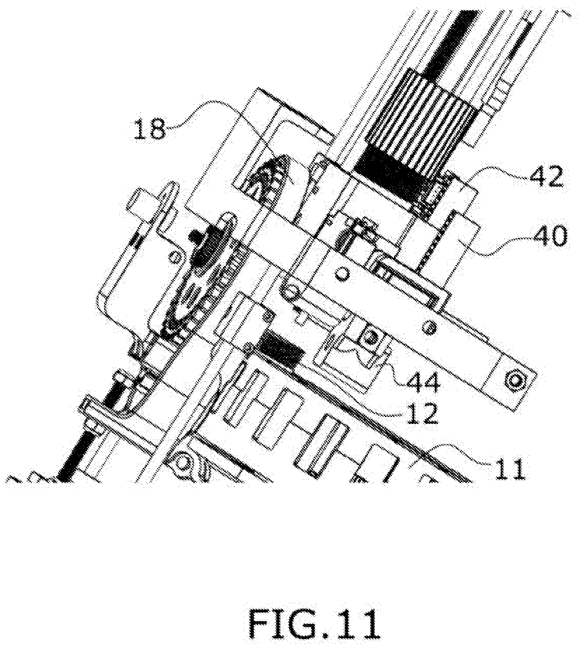

Figure 9, in addition to the elements described above, shows a first injection servo (40) associated with a first rack (41) and a second servo (42) associated with a second rack (43), wherein the assembly formed by the second servo (42) and the second rack (43) serves to be able to vertically move a secondary sensor (44) (figures 10 and 11) within the rotating and removable loader (11) and to be able to analyse the content of the samples stored within each container (12).

Having thus adequately described the nature of the present invention, as well as how to put it into practice, it must be noted that, within its essential nature, the invention may be carried out according to other embodiments differing in detail from that set out by way of example, which the protection sought would equally cover, provided that the fundamental principle thereof is not altered, changed or modified.

## Claims

1. Molecular spectroscopy equipment with a broad scanning range for continuously characterising the pollutant load of waste water, including characterising the size and mass concentration of particles, **characterised in that** it comprises:
- a first portion (2) arranged at the lower portion of the equipment and intended for the water pumping means,
- a main support platform (17) which is secured to the side walls of the equipment and which divides the interior space above the means intended for pumping water into a lower portion and an upper portion,
- an assembly for storing and analysing samples over time housed in the lower space defined under the main support platform (17) and attached to side walls of the equipment and comprising a rotating and removable loader (11) housing containers (12) for storing and analysing the samples, where this rotating and removable loader is provided with extraction and rotation means,
- a suction assembly (26) for taking samples mounted on a main block (33) for taking samples on which a primary sensor (31) is arranged, the main block being attached on the main support platform (17).
- Lifting means (34) for lifting the suction assembly (26) for taking samples arranged on the main block (33) for taking samples.
- A multispectral analysis unit (18) arranged opposite to the rotating and removable loader (11) and with the suction assembly (26) for taking samples, wherein said multispectral analysis unit is equipped with means that enable both a rotation and a lifting and lowering movement,
- means for injecting the samples taken arranged on the main support platform (17).
- Positioning means for positioning a secondary sensor mounted on the main block (33) for taking samples.
- An electronic panel (51) intended to support all the control and power supply means of the equipment.

2. The molecular spectroscopy equipment with a broad scanning range for continuously characterising the pollutant load of waste water, including characterising the size and mass concentration of particles, according to claim 1 **characterised in that** the extraction and rotation means of the rotating and removable loader (11) comprise a removable cart (13) on which the loader is mounted and which runs on a removable platform by means of telescopic rails (14) that are fastened to anchors (16) attached to the side walls of the interior of the equipment, wherein under the removable cart (13) a motor is housed responsible for rotating the rotating and removable loader (11).

3. The molecular spectroscopy equipment with a broad scanning range for continuously characterising the pollutant load of waste water, including characterising the size and mass concentration of particles, according to claim 1 or 2 **characterised in that** the suction assembly comprises a suction cylinder (27) made of transparent glass which is provided in its upper portion with a grooved upper cover (47) and on which is arranged a handle (28), wherein the suction cylinder (27) can be housed in an outer jacket (29) which is equipped with a window (30), said outer jacket (29) being housed within the main block (33) for taking samples so that the window (30) is aligned with the location of the primary sensor (31), a location being observed on the main block (33) for taking samples to house a lifting motor (32) that vertically moves the rod of the suction cylinder (27).

4. The molecular spectroscopy equipment with a broad scanning range for continuously characterising the pollutant load of waste water, including characterising the size and mass concentration of particles, according to claim 3 **characterised in that** the lifting and lowering means (34) of the mechanism for taking samples comprises a spindle (35) driven by the lifting motor (32), said spindle (35) being connected to a lifting platform (36) by means of a nut (48) which causes the transformation of the rotation of the spindle (35) into a vertical movement and wherein said lifting platform (36) comprises a connector (37) with the plunger (49) of the suction assembly.

5. The molecular spectroscopy equipment with a broad scanning range for continuously characterising the pollutant load of waste water, including characterising the size and mass concentration of particles, according to claim 4, **characterised in that** the vertical displacement of the lifting platform (36) is limited by a limit stop (38) and guided by means of guide rods (39) that are connected at their lower end to the main block (33) for taking samples and at their upper end they pass through the lifting platform (36).

6. The molecular spectroscopy equipment with a broad scanning range for continuously characterising the pollutant load of waste water, including characterising the size and mass concentration of particles, according to any of the preceding claims **characterised in that** the multispectral analysis unit (18) comprises a wavelength selector disc (19) which is a gear and on which is arranged a support plate (20) on which a plurality of LED diodes (21) are mounted such that the assembly rotates producing the alignment of each LED diode (21) with the sample located in the suction cylinder (27).

7. The molecular spectroscopy equipment with a broad scanning range for continuously characterising the pollutant load of waste water, including characterising the size and mass concentration of particles, according to claim 6 **characterised in that** the multispectral analysis unit (18) is equipped with two movements, a first lifting and lowering movement and a second rotation movement of the wavelength selector disc (19), wherein the first movement is achieved by means of a lifting and lowering motor (22), which is mounted on a support plate (50), wherein said motor rotates a threaded rod (23) which in its coupling with a support (45) of the wavelength selector disc (19) translates the rotation of the threaded rod (23) into a lifting and lowering movement and wherein said lifting and lowering movement is guided by means of vertical rods (24) on which slides (46) of the support (45) run, wherein said vertical rods (24) are attached to the lower side of the support plate (50) and at the upper side on the main support platform (17) and the rotation movement of the wavelength selector disc (19) is achieved by means of a motor (24) that has a pinion (25) coupled to its shaft that meshes with the wavelength selector disc (19).

8. The molecular spectroscopy equipment with a broad scanning range for continuously characterising the pollutant load of waste water, including characterising the size and mass concentration of particles, according to any of the preceding claims, **characterised in that** the distance between the secondary sensor and the containers (12) is the same as that between the main samples of the suction cylinder (27) and the primary sensor. Both make use of the wavelength selector disc (19), which after rotating 180° can align the same LED with the two different measurement devices provided in the equipment.

9. The molecular spectroscopy equipment with a broad scanning range for continuously characterising the pollutant load of waste water, including characterising the size and mass concentration of particles, according to any of the preceding claims, **characterised in that** the means for injecting the samples taken comprise a first injection servo (40) associated with a first rack (41).

10. The molecular spectroscopy equipment with a broad scanning range for continuously characterising the pollutant load of waste water, including characterising the size and mass concentration of particles, according to any of the preceding claims, **characterised in that** the positioning means for positioning a secondary sensor comprise a second servo (42) associated with a second rack (43).

11. The molecular spectroscopy equipment with a broad scanning range for continuously characterising the pollutant load of waste water, including characterising the size and mass concentration of particles, according to any of the preceding claims, **characterised in that** the rotating and removable loader (11) can be changed for another that allows holding containers having a different volume and number depending on the user's requirements.
